# EUROPEAN PATENT APPLICATION

(11) **EP 3 581 658 A1**
(43) Date of publication of application: **18.12.2019**
(21) Application number: 18751518.4
(22) Date of filing: 06.02.2018
(51) Int. Cl.: C12P 7/06, B01D 61/14, C12M 1/00

(54) **METHOD FOR PRODUCING ALCOHOL BY CONTINUOUS FERMENTATION AND CONTINUOUS FERMENTATION APPARATUS TO BE USED THEREFOR**

(30) Priority: 07.02.2017 JP 2017020394
(71) Applicant: Toray Industries, Inc., Tokyo 103-8666 (JP)
(72) Inventor: MIMITSUKA Takashi, Kamakura-shi Kanagawa 248-8555 (JP); SUDA Kazumi, Kamakura-shi Kanagawa 248-8555 (JP); HIGASA Masashi, Kamakura-shi Kanagawa 248-8555 (JP)
(74) Representative: Kador & Partner PartG mbB
(86) International application number: PCT/JP2018/004087
(87) International publication number: WO 2018/147289

(57) **Abstract**

Provided is a method for producing an alcohol by continuous fermentation using a fermentation raw material, the method comprising steps of: culturing a microorganism in the fermentation raw material in a fermenter to obtain a fermented liquid, while supplying the fermentation raw material into the fermenter using a plurality of fermentation raw material supply systems; filtering the obtained fermented liquid through a separation membrane module to obtain an alcohol-containing filtered liquid and an unfiltered liquid; and retaining or refluxing the obtained unfiltered liquid in the fermenter; in which the plurality of fermentation raw material supply systems each independently comprise a fermentation raw material tank for storing the fermentation raw material which is the same as each other, and a supply line connecting the fermentation raw material tank and the fermenter; and wherein the method comprises selecting a fermentation raw material supply system to be allowed to stop supplying the fermentation raw material in rotation from the plurality of fermentation raw material supply systems during the supply of the fermentation raw material using the plurality of fermentation raw material supply systems.

## Description

### TECHNICAL FIELD

The present invention relates to a method for producing an alcohol by continuous fermentation, and a continuous fermentation apparatus to be used therefor.

### BACKGROUND OF THE INVENTION

Fermentation methods, which are substance production methods involving cultivation of microorganisms or cultured cells, can be largely classified to (1) a batch fermentation method and a fed-batch fermentation method, and (2) a continuous fermentation method.

The batch fermentation and the fed-batch fermentation method have the advantages of simple equipment, short time to complete cultivation, and less damage from contamination. However, since the concentration of products in the culture increases with time, the productivity and yield decrease due to the influence of osmotic pressure, product inhibition, or the like. Because of this, it is difficult to stably keep the yield and productivity high for a long period of time.

On the other hand, the continuous fermentation method is performed by continuously culturing microorganisms or cultured cells in a fermenter while continuously supplying a culture medium to the fermenter. For example, a continuous culture method used for fermentation of L-glutamic acid or L-lysine has been disclosed (Non-patent Document 1).

Such a continuous fermentation method should theoretically enable a long-term efficient production of target substances. However, despite such an advantage, it is considered practically difficult to use conventional continuous fermentation methods to realize a long-term continuous operation on an industrial scale due to an increase in contamination risk in fermenters or the like (Non-patent Document 1).

Furthermore, in continuous culture methods, a culture containing microorganisms for use in fermentation is remove from the fermenter while a fermentation raw material is continuously supplied to the fermenter, resulting in dilution of the microorganisms in the culture, which may limit the improvement of the production efficiency. Thus, maintaining the concentration of microorganisms in the culture at high level in continuous fermentation methods has been an important issue in the continuous fermentation field.

As a means to solve the above problems, the present inventors have proposed a continuous fermentation method including filtering a fermented liquid containing a microorganism through a separation membrane, recovering a product from the filtrate while retaining or refluxing the filtered microorganism in the culture to maintain the concentration of the microorganism in the fermenter at a high level, and a continuous fermentation apparatus for use in the method (Patent Document 1).

### PRIOR ART DOCUMENTS

### PATENT DOCUMENT

Patent Document 1: WO2007/097260

### NON-PATENT DOCUMENTS

Non-patent Document 1: Toshihiko Hirao et. al., Appl. Microbiol. Biotechnol., 32, 269-273
Non-patent Document 2: P.F. Strambury, and A. Whitaker, "Principles of Fermentation Technology, From Laboratory to Industrial Scale", Gakkai Publication Center, Sep. 1, 1998 (1st edition)

### SUMMARY OF THE INVENTION

### PROBLEMS TO BE SOLVED BY THE INVENTION

A continuous fermentation apparatus wherein a microorganism and the like are retained or refluxed in a fermenter by means of a separation membrane can efficiently utilize the microorganism, and thus is expected to enable a longer-term continuous fermentation than conventional continuous fermentation apparatus. The present inventors, as shown in Examples 1 and 2 later, examined how long production of an alcohol by continuous fermentation can be continued using a continuous fermentation apparatus wherein a microorganism and the like were retained or refluxed in a fermenter by means of a separation membrane and wherein a raw material and the apparatus were not sterilized, and revealed that only after about 300 hours a decrease in the fermentation efficiency due to contamination was observed. Thus, it is also important issues in a continuous fermentation apparatus wherein a microorganism and the like are retained or refluxed in a fermenter by means of a separation membrane, to prevent contamination and continue alcohol production by continuous fermentation for a long period of time.

In order to solve the above problem, the present inventors have intensively studied to find that a continuous fermentation apparatus wherein a microorganism is retained or refluxed in a fermenter by using a separation membrane and wherein a plurality of independent fermentation raw material supply systems are used in rotation to supply a fermentation raw material to a fermenter can effectively prevent contamination and efficiently produce an alcohol by continuous fermentation for a long period of time. The present invention is based on the findings.

### MEANS FOR SOLVING THE PROBLEMS

In accordance with the present invention, there are provided:
(1) A method for producing an alcohol by continuous fermentation using a fermentation raw material,
   the method comprising steps of: culturing a microorganism in the fermentation raw material in a fermenter to obtain a fermented liquid, while supplying the fermentation raw material into the fermenter using a plurality of fermentation raw material supply systems; filtering the obtained fermented liquid through a separation membrane module to obtain an alcohol-containing filtered liquid and an unfiltered liquid; and retaining or refluxing the obtained unfiltered liquid in the fermenter;
   wherein the plurality of fermentation raw material supply systems each independently comprise a fermentation raw material tank for storing the fermentation raw material which is the same as each other, and a supply line connecting the fermentation raw material tank and the fermenter; and
   wherein the method comprises selecting a fermentation raw material supply system to be allowed to stop supplying the fermentation raw material in rotation from the plurality of fermentation raw material supply systems during the supply of the fermentation raw material using the plurality of fermentation raw material supply systems.
(2) The production method according to (1), wherein the fermentation raw material is not sterilized.
(3) The production method according to (1) or (2), wherein each of the fermentation raw material supply systems stops supplying the fermentation raw material each time the system supplies the fermentation raw material for 1 to 6 days.
(4) The production method according to any one of (1) to (3), wherein either of the fermentation raw material supply systems is washed while stopping the supply of the fermentation raw material.
(5) The production method according to (4), wherein the fermentation raw material supply system stopping the supply of the fermentation raw material resume the supply of the fermentation raw material after the washing.
(6) The production method according to (4) or (5), comprising supplying a liquid obtained through the washing of the fermentation raw material supply system to the fermenter.
(7) The production method according to any one of (1) to (6), wherein the alcohol is at least one selected from the group consisting of methanol, ethanol, propanol and butanol; .
(8) The production method according to any one of (1) to (7), wherein the fermentation raw material is molasses.
(9) A continuous fermentation apparatus for producing an alcohol by continuous fermentation using a fermentation raw material, the apparatus comprising:
   a plurality of fermentation raw material supply systems for supplying the fermentation raw materials which are the same as each other;
   a fermenter in which a microorganism is cultured in the fermentation raw material supplied from the plurality of fermentation raw material supply systems to obtain a fermented liquid;
   a separation membrane module for filtering the fermented liquid obtained in the fermenter;
   a first line connecting the fermenter and the separation membrane module and delivering the fermented liquid obtained in the fermenter to the separation membrane module; and
   a second line connecting the separation membrane module and the fermenter and delivering an unfiltered liquid obtained by filtration of the fermented liquid in the separation membrane module to the fermenter;
   wherein the plurality of fermentation raw material supply systems each independently comprise a fermentation raw material tank for storing the fermentation raw material which contains sugars and is the same as each other, and a supply line connecting the fermentation raw material tank and the fermenter.
(10) The continuous fermentation apparatus according to (9), wherein the supply line comprises a line mixer.
(11) The continuous fermentation apparatus according to (9) or (10), wherein the fermentation raw material supply system does not comprise a sterilizer for the fermentation raw material.
(12) The continuous fermentation apparatus according to any one of (9) to (11), wherein the apparatus further comprises a washing liquid tank connected to the plurality of fermentation raw material supply systems.
(13) The continuous fermentation apparatus according to (12), wherein the apparatus further comprises washing liquid tanks each connected to each of the plurality of fermentation raw material supply systems.

### EFFECT OF THE INVENTION

In accordance with the present invention, a continuous fermentation apparatus characterized by retaining or refluxing a microorganism and the like in a fermenter while using a separation membrane allows for effective prevention of contamination, and efficient production of an alcohol by continuous fermentation for a long period of time.

### BRIEF DESCRIPTION OF THE DRAWINGS

FIG. 1 is a schematic view showing one embodiment of the continuous fermentation apparatus of the present invention.
FIG. 2 is a schematic view showing a continuous fermentation apparatus used in Examples 1 to 3.
FIG. 3 is a graph showing change in the concentration of an alcohol in a filtrate obtained through a continuous fermentation in Example 1 over time.
FIG. 4 is a graph showing change in the concentrations of glucose, fructose, and lactic acid, as well as in pH, in a filtrate obtained through a continuous fermentation in Example 1 over time.
FIG. 5 is a graph showing change in the concentrations of glucose, fructose, and lactic acid, as well as in pH, in a filtrate obtained through a continuous fermentation in Example 2 over time.
FIG. 6 is a graph showing change in the concentration of an alcohol in a filtrate obtained through a continuous fermentation in Example 3 over time.
FIG. 7 is a graph showing change in the concentrations of glucose, fructose, and lactic acid, as well as in pH, in a filtrate obtained through a continuous fermentation in Example 3 over time.

### DETAILED DESCRIPTION OF THE INVENTION

### Method for Producing Alcohol by Continuous Fermentation

The method of the present invention for producing an alcohol by continuous fermentation using a fermentation raw material comprises steps of:
culturing a microorganism in the fermentation raw material in a fermenter to obtain a fermented liquid, while supplying the fermentation raw material into the fermenter using a plurality of fermentation raw material supply systems; filtering the obtained fermented liquid through a separation membrane module to obtain an alcohol-containing filtered liquid and an unfiltered liquid; and retaining or refluxing the obtained unfiltered liquid in the fermenter;
wherein the plurality of fermentation raw material supply systems each independently comprise a fermentation raw material tank for storing the fermentation raw material which is the same as each other, and a supply line connecting the fermentation raw material tank and the fermenter; and
wherein the method comprises selecting a fermentation raw material supply system to be allowed to stop supplying the fermentation raw material in rotation from the plurality of fermentation raw material supply systems during the supply of the fermentation raw material using the plurality of fermentation raw material supply systems.

### Step of Providing Fermentation Raw Material

In the method for producing an alcohol in the present invention, the fermentation raw material is firstly provided.

Preferred examples of the fermentation raw material in the present invention include common liquid media containing organic micronutrients such as carbon sources, nitrogen sources, and inorganic salts as appropriate. The carbon sources that can be used include sugars such as glucose, sucrose, fructose, galactose, and lactose; starch saccharified solutions containing the sugars, sweet potato molasses, sugar beet molasses, syrup (molasses), Hi-test molasses, and cane molasses; organic acids; and glycerins. The nitrogen sources that can be used include ammonia gas, aqueous ammonia, ammonium salts, urea, nitrates, other supplementary added organic nitrogen sources, for example, oil cakes, soybean hydrolysates, casein digests, other amino acids, vitamins, corn steep liquors, yeast or yeast extracts, meat extracts, peptides such as peptone, various fermentation microorganisms and hydrolysates thereof. Inorganic salts such as phosphates, magnesium salts, calcium salts, iron salts, and manganese salts can be added as appropriate.

As the fermentation raw material in the present invention, a non-sterilized non-sterile fermentation raw material is preferably used so as not to destroy nutrients in the fermentation raw material by heat. As used herein, the term "sterilization" means a process for completely destroying or eliminating proliferative microorganisms. Specific examples of the process include a process for killing microorganisms by keeping a fermentation raw material under a high temperature condition of 100°C or higher or under high-temperature and high-pressure conditions; a process for removing microorganisms by filtering a fermentation raw material; a process for making the proliferative ability of microorganisms lost through irradiation of a fermentation raw material with UV, gamma-ray, or the like. As the non-sterile fermentation raw material in the present invention, a fermentation raw material which are not treated with the sterilization as described above is suitably used. The non-sterile fermentation raw material in the present invention may be in the state where proliferative microorganisms are not completely destroyed or eliminated. Such a non-sterile fermentation raw material is preferably a fermentation raw material containing sugar, more preferably a fermentation raw material containing syrup (molasses) as a main component. As used herein, the term "a fermentation raw material containing molasses as a main component" means that 50 percent by weight or more of substances contained in the fermentation raw material (except water) is molasses. Continuous fermentation can be advantageously performed using a non-sterile fermentation raw material as in the present invention for a long period of time from the viewpoint of industrial production efficiency. In particular, a fermentation raw material containing a high concentration of sugar including molasses can be preferably used for continuing a continuous fermentation for a long period of time because microorganisms hardly propagate in the fermentation raw material.

### Step of Producing Alcohol

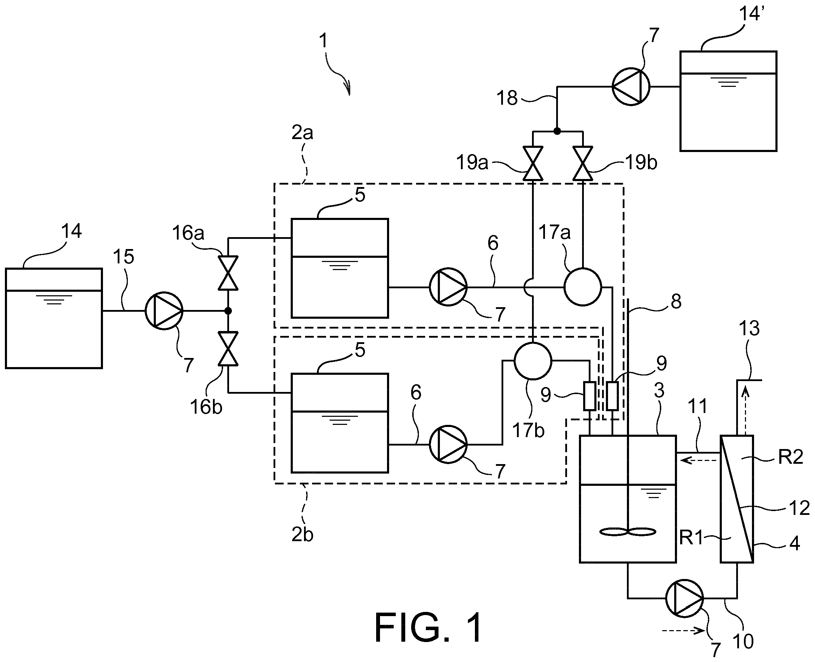

The method for producing an alcohol of the present invention comprises culturing a microorganism in the fermentation raw material as described above in a fermenter to obtain a fermented liquid while supplying the fermentation raw material into the fermenter using a plurality of fermentation raw material supply systems; filtering the obtained fermented liquid through a separation membrane module to obtain an alcohol-containing filtered liquid and an unfiltered liquid; and retaining or refluxing the obtained unfiltered liquid in the fermenter. The method for producing an alcohol of the present invention will now be described in more detail with reference to FIG. 1.

FIG. 1 is a schematic view showing one embodiment of the continuous fermentation apparatus for use in the method for producing an alcohol of the present invention. The continuous fermentation apparatus (1) shown in FIG. 1 comprises a plurality of fermentation raw material supply systems (2), a fermenter (3), and a separation membrane module (4).

### [Supply of the Fermentation Raw Material Using a Plurality of Fermentation Raw Material Supply Systems]

In the method for producing an alcohol of the present invention, the fermentation raw material is supplied to the fermenter (3) using the plurality of fermentation raw material supply systems (2a and 2b). The plurality of fermentation raw material supply systems (2a and 2b) each independently comprise a fermentation raw material tank (5) for storing the fermentation raw material, and a supply line (6) connecting the fermentation raw material tank (5) and the fermenter (3). Preferably, a pump (7) is disposed in the supply line (6) for delivering the fermentation raw material to the fermenter (3). The plurality of fermentation raw material supply systems (2a and 2b) supply the same fermentation raw material to the fermenter (3). The number of the fermentation raw material supply system in the present invention is not particularly limited and may be two or more, for example, three to five, and may be set as appropriate by those skilled in the art in consideration of fermentation efficiency and the like.

In some embodiments of the present invention, the production method comprises stopping one of the fermentation raw material supply systems (2a and 2b) from supplying the fermentation raw material during supply of the fermentation raw material using the plurality of fermentation raw material supply systems (2a and 2b). The fermentation raw material supply system to be allowed to stop supplying the fermentation raw material is selected from the plurality of fermentation raw material supply systems (2a and 2b) in rotation. For example, when the fermentation raw material supply system (2a) is stopped, the fermentation raw material supply system (2b) is operated to supply the fermentation raw material. On the other hand, when the fermentation raw material supply system (2b) is stopped, the fermentation raw material supply system (2a) is operated to supply the fermentation raw material. In continuous fermentation processes, the rate of supply of a fermentation raw material is preferably constant. Thus, regardless of the number of the fermentation raw material supply systems in operation, the total rate of supply of the fermentation raw material supplied from the fermentation raw material supply systems is preferably regulated to be constant. Thus, for easy regulation, one fermentation raw material supply system is preferably in operation. Preferably, the fermentation raw material tank (5) preferably contains a high concentration of the fermentation raw material. Thus, the fermentation raw material is preferably diluted into an appropriate sugar concentration with water just before being supplied to the fermenter. Thus, the supply lines (6) in the fermentation raw material supply systems (2a and 2b) are preferably provided with line mixers (17a and 17b), respectively. In addition, a water tank (14') is also preferably disposed in order to supply water to the line mixers (17a and 17b). The water tank (14') may be connected to the line mixers (17a and 17b), for example, through a line (18) having a T-branch as shown in FIG. 1. The line (18) may be provided with connecting valves (19a and 19b) so that the supply destination of water can be selected by their opening and closing. Further, a pump (7) may also be disposed to deliver water to the line mixers (17a and 17b).

Either of the fermentation raw material supply systems (2a and 2b) is washed while stopping the supply of the fermentation raw material. Surprisingly, as described above, washing of fermentation raw material systems while stopping them in rotation at an appropriate interval described below enables a continuous fermentation to be continued for a long period of time without sterilization of a fermentation raw material.

For the fermentation raw material supply systems (2a and 2b) in the present invention, the interval at which the supply of the fermentation raw material is stopped is determined as appropriate by those skilled in the art depending on the type of bacteria, temperature, humidity, scale, and the like. For example, the interval is from 1 to 10 days, preferably from 1 to 6 days, more preferably from 1 to 2 days.

### [Washing of the Plurality of Fermentation Raw Material Supply Systems]

As shown in FIG. 1, the plurality of fermentation raw material supply systems (2a and 2b) are preferably connected, through a line, to a washing liquid tank (14) containing a washing liquid (such as water) for washing the inside of the system. In FIG. 1, the washing liquid tank (14) is connected to the fermentation raw material tanks (5) in the fermentation raw material supply systems (2a and 2b) through a line (15) having a T-branch. Preferably, the fermentation raw material tank (5) is connected to a spray ball provided in the top plate part inside the tank. The line (15) is provided with connecting valves (16a and 16b), as well as with a pump (7) for delivering the washing liquid to the fermenter (2).

When the fermentation raw material supply system (2a) is stopped, the connecting valve (16a) can be opened to wash the fermentation raw material tank (5) and the line (6) in the fermentation raw material supply system (2a) with the washing liquid. When the fermentation raw material supply system (2b) is stopped, the connecting valve (16b) can be opened to wash the fermentation raw material tank (5) and the line (6) in the fermentation raw material supply system (2b) with the washing liquid. The number of the washing liquid tank connected to the fermenter is not particularly limited and may be one or two or more. From the viewpoint of equipment cost, one washing liquid tank is preferred as shown in FIG. 1.

When the fermentation raw material supply system (2a or 2b) is washed, the liquid obtained by washing the fermentation raw material supply system (2a or 2b) is preferably supplied to the fermenter (3). The liquid obtained by the washing, which contains in solution the fermentation raw material remaining in the fermentation raw material tank (5) and the supply line (6), is preferably supplied to the fermenter from the viewpoint of efficient use of the fermentation raw material.

Preferably, the supply lines (6) in the fermentation raw material supply systems (2a and 2b) are each provided with a sugar concentration meter (9) in order to check whether the fermentation raw material is diluted by the line mixer according to the setting or to measure the concentration of the remaining fermentation raw material in the washing liquid. The sugar concentration meter (9) may be placed at any particular position in the supply line (6), preferably near the junction between the supply line (6) and the fermenter (3) from the viewpoint of accurate measurement of the sugar concentration in the fermentation raw material.

### [Fermentation Using Fermenter and Separation Membrane Module]

The method for producing an alcohol of the present invention comprises culturing a microorganism in the fermentation raw material in the fermenter using the fermentation raw material supplied from the plurality of fermentation raw material supply systems as described above to obtain a fermented liquid; filtering the obtained fermented liquid through a separation membrane module to obtain an alcohol-containing filtered liquid and an unfiltered liquid; and retaining or refluxing the obtained unfiltered liquid in the fermenter.

As shown in FIG. 1, the fermenter (3) is provided with a stirrer (8) for stirring the fermentation raw material and a microorganism in the fermenter (3). In the fermenter (3), the microorganism is cultured in the fermentation raw material supplied from a plurality of fermentation raw material supply systems (2) to obtain a fermented liquid.

Any number of fermenters may be employed in the production method of the present invention without impairing the effects of the present invention. In the production method of the present invention, the continuous culture operation is usually and preferably performed in a single fermenter as shown in FIG. 1 from the viewpoint of culture control. However, from reasons such as small volume of the fermenter, a plurality of fermenters can be used. For example, a plurality of fermenters may be connected in parallel or in series through pipes for performing the continuous culture.

In the production method of the present invention, the concentration of sugars in the culture is preferably maintained at 5 g/L or less. The concentration of sugars in the culture is preferably maintained at 5 g/L or less in order to minimize the loss of sugars by removing of the culture. The concentration of sugars in the culture is adjusted as appropriate according to the composition or the supply rate of the fermentation raw material supplied from the fermentation raw material supply system.

The microorganism can usually be cultured in a pH range from 4 to 8 and in a temperature range from 20 to 40°C. The pH of the culture can be adjusted to a predetermined value usually in pH range from 4 to 8 with an inorganic or organic acid, or an alkaline substance, or even urea, calcium carbonate, ammonia gas, or the like. Further, aeration may be performed as needed.

In the production method of the present invention, a batch culture or fed-batch culture may be performed early in the cultivation to increase the concentration of the microorganism before the start of the continuous culture. In the production method of the present invention, the concentration of the microorganism may be increased, then the increased concentration of the microorganism may be seeded, and thereafter the continuous culture may be performed at the start of the cultivation. In the production method of the present invention, supply of the fermentation raw material and continuous culture of the culture can be started at appropriate times. The supply of the fermentation raw material and the continuous culture are not necessarily started at the same time.

In order to achieve efficient productivity, the concentration of the microorganism (including cultured cell) in the culture in the fermenter is preferably maintained in a high level, at which the environment of the culture is suitable for the growth of the microorganism and the ratio of death does not increase. As an example, the concentration of the microorganism in the culture can be maintained at 5 g/L or more by dry weight to obtain good production efficiency.

In the production method of the present invention, the microorganism can be removed from the fermenter as needed. For example, since the separation membrane tends to be clogged when the concentration of the microorganism in the fermenter is too high, the clogging can be avoided by removing the microorganism.

Next, microorganisms that can be used in the production method of the present invention are described below. The microorganism used in the production method of the present invention is not limited as long as it can be used for the alcohol production. Examples of the microorganism that can be used for the alcohol production include yeasts belonging to the Genus Saccharomyces, Genus Kluyveromyces, and Genus Schizosaccharomyces. Among them, Saccharomyces cerevisiae, Kluyveromyces lactis, and Schizosaccharomyces pombe can be suitably used.

The microorganism that can be used for production of the alcohol production in the present invention may be a microorganism or a cultured cell having an artificially enhanced capability for producing an alcohol. Specifically, the microorganism that can be used for production of the alcohol production in the present invention may be a microorganism having characteristics partially modified by mutation or gene modification. One example of the microorganism having partially modified characteristics include a yeast that obtains an ability to metabolize raw starch by introduction of a glucoamylase gene of a mold belonging to the Genus Rhizopus (Microorganisms, 3: 555-564(1987)).

### [Separation of Alcohol-Containing Filtered Liquid and Unfiltered Liquid in Separation Membrane Module]

In some embodiments of the present invention, the fermented liquid obtained as described above is filtered through a separation membrane module to obtain an alcohol-containing filtered liquid and an unfiltered liquid, and then the obtained unfiltered liquid is retained or refluxed in the fermenter. The microorganism and the like contained in the unfiltered liquid can be retained or refluxed in the fermenter as described above to perform an efficient alcohol fermentation.

As shown in FIG. 1, the fermenter (3) is connected to the separation membrane module (4) via the first line (10), and a fermented liquid obtained in the fermenter (3) is delivered to the separation membrane module (4) through the first line (10). The first line (10) may be provided with a pump (7) for delivering a fermented liquid obtained in the fermenter (3) to the separation membrane module (4).

The fermenter (3) is also connected to the separation membrane module (4) via the second line (11), and an unfiltered liquid (for example, larger matters than the pore size of the porous membrane (12), such as microorganism) obtained by filtering the fermented liquid through the separation membrane module (4) is delivered to the fermenter (3) through the second line (11). The second line (11) may be provided with a pump (7) for delivering a fermented liquid obtained in the fermenter (3) to the separation membrane module (4).

The separation membrane module (4) has a substantially cylindrical shape, and has a porous membrane (12) inside. The porous membrane (12) is arranged so that the interior space of the separation membrane module (4) is divided to form two regions, R1 and R2. Thus, the separation membrane module (4) is in a state where the interior space is divided by the porous membrane (12) and the two divided spaces are communicated with each other only through the pores. In the separation membrane module (4), the first line (10) and the second line (11) are joined to communicate with the region R1, while a third line (13) is joined to communicate with the region R2.

In separation membrane module (4), the fermented liquid delivered from the fermenter (3) is filtered through the porous membrane (12) and separated into an alcohol-containing filtered liquid and an unfiltered liquid. Specifically, the alcohol-containing filtered liquid that has passed through the pores of the porous membrane (12) enters the region R2 and is delivered outside through the third line (13). On the other hand, the unfiltered liquid that has not passed through the pores of the porous membrane (12) remains in the region R1, refluxed to the fermenter (3) through the second line (11), and held in the fermenter (3). As described above, the fermenter (3) is subjected to supply of the fermentation raw material from the raw material supply tank (5) and the reflux of the microorganism and the like from the separation membrane module (4) repeatedly, which enables an efficient continuous fermentation.

The separation membrane module (4) may comprise the porous membrane (12) described above to divide the interior space, or comprise a hollow fiber separation membrane module comprising a plurality of hollow fiber membrane. When hollow fiber separation membrane modules are used, for example, when an external pressure hollow fiber membrane module is used, the first line (10) and the second line (12) are joined to the module to communicate with a space formed on the side of the outer surface of the hollow fiber membrane (corresponding to the region R1), while the third line (13) is joined to the module to communicate with a space formed on the side of the inner surface of the hollow fiber membrane (corresponding to the region R2). When an internal pressure hollow fiber membrane module is used, the first line (10) and the second line (12) are joined to the module to communicate with a space formed on the side of the inner surface of the hollow fiber membrane, while the third line (13) is joined to the module to communicate with a space formed on the side of outer surface of the hollow fiber membrane.

From the alcohol-containing filtered liquid obtained by the production method of the present invention, an alcohol can be suitably separated and purified by, for example, purification via distillation, or purification via concentration using an NF membrane, an RO membrane, or a zeolite separation membrane.

The concentration of an alcohol in the alcohol-containing filtered liquid is not particularly limited, and can be adjusted according to fermentation conditions, the rate of filtration, and the like, and can be within a range, for example, from 30 to 120 g/L.

Preferred examples of the alcohol obtained by the production method of the present invention include methanol, ethanol, propanol, butanol and combinations thereof. Preferably, the alcohol is ethanol.

The production method of the present invention can also prevent any contamination to advantageously reduce the pH decrease in the culture in the fermenter. The pH of the culture in the fermenter during the continuous fermentation is, for example, from about 3 to 8, preferably from about 4 to 6.

The production method of the present invention enables a long-term continuous fermentation even using a non-sterile fermentation raw material. The period for performing the continuous fermentation according to the present invention is not particularly limited. The suitable lower limit can be selected from, for example, 100 hours, 200 hours, 300 hours, 400 hours, 900 hours and 1,000 hours. The suitable upper limit of the period for performing the continuous fermentation can be selected from, for example, 1,000 hours, 1,500 hours, and 2,000 hours. Particularly suitable period for performing the continuous fermentation can be, for example, 100 to 2,000 hours.

### Continuous Fermentation Apparatus

In accordance with the present invention, the continuous fermentation apparatus as illustrated in FIG. 1 can be used to efficiently produce an alcohol. Thus, according to another aspect of the present invention, there is provided a continuous fermentation apparatus for producing an alcohol by continuous fermentation using a fermentation raw material, the apparatus comprising:
a plurality of fermentation raw material supply systems for supplying the fermentation raw materials which are the same as each other;
a fermenter in which a microorganism is cultured in the fermentation raw material supplied from the plurality of fermentation raw material supply systems to obtain a fermented liquid;
a separation membrane module for filtering the fermented liquid obtained in the fermenter;
a first line connecting the fermenter and the separation membrane module and delivering the fermented liquid obtained in the fermenter to the separation membrane module; and
a second line connecting the separation membrane module and the fermenter and delivering an unfiltered liquid obtained by filtration of the fermented liquid in the separation membrane module to the fermenter;
wherein the plurality of fermentation raw material supply systems each independently comprise a fermentation raw material tank for storing the fermentation raw material which comprises sugars and is the same as each other, and a supply line connecting the fermentation raw material tank and the fermenter.

Preferably, the continuous fermentation apparatus of the present invention comprises a washing liquid tank and/or a line mixer as shown in FIG. 1. In a preferred embodiment, the line mixer is disposed in the supply line. In the continuous fermentation apparatus of the present invention, one washing liquid tank is preferably connected to the plurality of fermentation raw material supply systems via a line from the viewpoint of efficient washing.

In the continuous fermentation apparatus of the present invention, the washing liquid tank, the lines (such as supply line, first line, and second line) and the fermenter can be a known tank, pipe and fermenter, respectively, depending on the desired size and characteristics of the fermentation raw material.

In the continuous fermentation apparatus of the present invention, the separation membrane module has a substantially cylindrical shape as illustrated in FIG. 1, and has inside a porous membrane in which a plurality of pores having a mean size of, for example, 0.01 µm or more and less than 1.00 µm are formed. Here, the mean pore size can be determined by measuring and averaging the diameters of all observable pores in an area of 9.2 µm × 10.4 µm in scanning electron microscopy at a magnification of x10,000. Alternatively, the mean pore size is determined by taking a photograph of the membrane surface using a scanning electron microscope at a magnification of x10,000, selecting 10 or more, preferably 20 or more pores at random, measuring the diameters of the pores, and calculating the number average. When the pore is not circular, a method can be used, in which a circle having the same area as that of the pore (equivalent circle) is obtained using an image processor or the like, and the diameter of the equivalent circle is considered as the diameter of the pore. The separation membrane module may be arranged to divide the interior space into two regions R1 and R2 as described above, or a hollow fiber membrane may be used.

The porous membrane or the hollow fiber membrane may be composed of organic materials and/or inorganic materials. Preferably, the organic materials are organic polymers, including polyethylene resins, polypropylene resins, polyvinyl chloride resins, polyvinylidene difluoride resins, polysulfone resins, polyether sulfone resins, polyacrylonitrile resins, cellulose resins and cellulose triacetate resins. Among them, from the viewpoint of ease of film formation with the solution and excellent physical durability and chemical resistance, polyvinyl chloride resins, polyvinylidene difluoride resins, polysulfone resins, polyether sulfone resins and polyacrylonitrile resins are preferred. Particularly preferably, a polyvinylidene difluoride resin or a resin mainly containing it is used. Preferred inorganic membranes include ceramics.

The continuous fermentation apparatus of the present invention may also be provided with sugar concentration meters, thermometers, and pH meters at desired positions in, for example, the fermenter and the lines, from the viewpoint of monitoring the progress of the fermentation and washing. The lines may also be provided with a valve for controlling the direction of liquid delivery or washing at a desired position.

The continuous fermentation apparatus of the present invention may also be provided with a control unit for controlling the stop duration of the fermentation raw material supply system, the washing duration, the rates of delivering liquids, and the like. For example, the control unit can store information from the sugar concentration meters, the thermometers, and the pH meters deposited in the continuous fermentation apparatus, and based on the information, can control the stop duration of the fermentation raw material supply system, the washing duration, the rates of delivering liquids and the like. The control unit can be provided by using a semiconductor memory such as a flash memory or a DRAM (Dynamic Random Access Memory).

In some embodiments of the present invention, a fermentation raw material supply system that does not comprise a fermentation raw material sterilizer can be used. The term "a fermentation raw material supply system that does not comprise a fermentation raw material sterilizer" refers to a fermentation raw material supply system that does not comprise any device for sterilizing the above-described fermentation raw material. Examples of the fermentation raw material sterilizer include tanks capable of retaining the fermentation raw material at a high temperature or at a high temperature and high pressure; continuous sterilizers capable of continuously performing a high-temperature treatment or a high-pressure treatment; filter devices for removing the microorganism from the fermentation raw material; and devices for irradiating the fermentation raw material with UV or gamma-ray. The fermentation raw material supply system that does not comprise a fermentation raw material sterilizer not only is more convenient and low-cost than the fermentation raw material supply system that comprises a fermentation raw material sterilizer, but also eliminates the need for sterilization management of the fermentation raw material supply system, and therefore is advantageous in reducing the management burden.

### EXAMPLES

The method of the present invention will now be described in more detail with reference to the Examples. Unless otherwise stated, the unit and the measuring method are in accordance with the provisions of the Japanese Industrial Standard (JIS).

### Example 1: Examination of Possible Duration of Continuous Fermentation in Case of No Sterilization

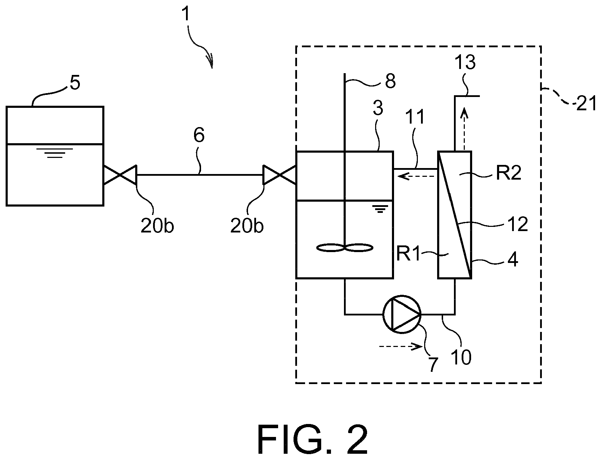

In order to determine the possible duration of continuous fermentation when a continuous fermentation apparatus comprising a separation membrane module is used to produce an alcohol without sterilization of a fermentation raw material and the equipment, the continuous fermentation apparatus (1) shown in FIG. 2 was operated for 497 hours. A fermentation raw material obtained by adding a bagasse-derived sugar solution prepared by a method described in WO2013/172446 to 5% to a molasses (Showa Sugar Co. Ltd.) was used. As indices of the continuous fermentation efficiency, the concentration of ethanol, the concentration of glucose (fermentation raw material), the concentration of fructose (fermentation raw material), and lactic acid in the filtrate obtained from the separation membrane module, and the pH value of the culture in the fermenter were used. The concentrations were measured over time during the examination.

### [Continuous Fermentation Apparatus]

The continuous fermentation apparatus (1) shown in FIG. 2 comprises the same fermenter (3) and separation membrane module (4) as of the continuous fermentation apparatus shown in FIG. 1, except for the fermentation raw material supply system. In the continuous fermentation apparatus (1) shown in FIG. 1, the fermentation raw material supply system comprises one fermentation raw material tank (5) and a supply line (6). In addition, the junction between the fermentation raw material tank (5) and the supply line (6) is provided with a connecting valve (20a), while the junction between the fermenter (3) and the supply line (6) is provided with a connecting valve (20b). The supply line (6) can be disconnected at the connecting valves (20a and 20b).

### [Conditions of Continuous Fermentation]

The conditions of continuous fermentation using the continuous fermentation apparatus (1) were as follows:
Fermentation raw material supply rate: 2 L/day
Microorganism for fermentation: Schizosaccharomyces pombe NBRC 1628
Amount of inoculated microorganism for fermentation: 5% inoculation
Fermentation conditions: at 30°C, without neutralization nor aeration
Stirring speed: 300 rpm
Conditions of use of separation membrane: membrane surface linear velocity 30 cm/s; filtration flux 0.1 m/d; a cycle of filtration for 9 min and stop of filtration for 1 min.

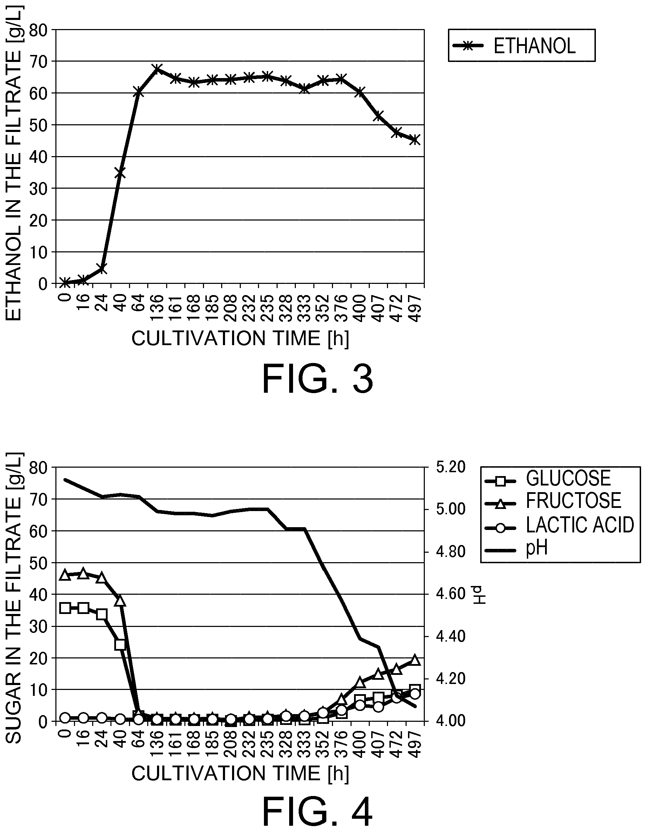

As a result of the continuous fermentation, the concentration of ethanol in the filtrate was as shown in FIG. 3. The concentration of ethanol in the filtrate decreased from 276 hours after the start of the fermentation.

On the other hand, the concentration of glucose, the concentration of fructose, and the concentration of lactic acid in the filtrate, and the pH value of the culture in the fermenter were as shown in FIG. 4. The concentrations of glucose and fructose derived from the fermentation raw material became almost zero at 64 hours after the start of the continuous fermentation, but then increased after about 300 hours from the start of the test. The concentration of lactic acid also increased after about 300 hours from the start of the test as did the concentrations of glucose and fructose. Further, the pH value of the culture in the fermenter also decreased after about 300 hours from the start of the test.

From the above results, it was found that the possible duration of continuous fermentation without sterilization of the fermentation raw material and the equipment using the continuous fermentation apparatus (1) shown in FIG. 2 was about 300 hours, and thereafter an increase in the concentration of lactic acid, lowering of the pH value, remaining of glucose and fructose derived from the fermentation raw material due to contamination were observed.

### Example 2: Fermentation with Sterilization of Fermentation Equipment

As described in Patent Document 1, it is known that there is a risk of contamination in continuous fermentation at the fermenter and the like. The region including the fermenter (3) enclosed in the dotted line (21) in the continuous fermentation apparatus (1) shown in FIG. 2 was sterilized in an autoclave at 121°C for 20 minutes, before continuous fermentation was carried out as in Example 1. In the test, as indices of contamination, the concentration of glucose, the concentration of fructose, and the concentration of lactic acid in the filtrate, and the pH value of the culture in the fermenter were measured.

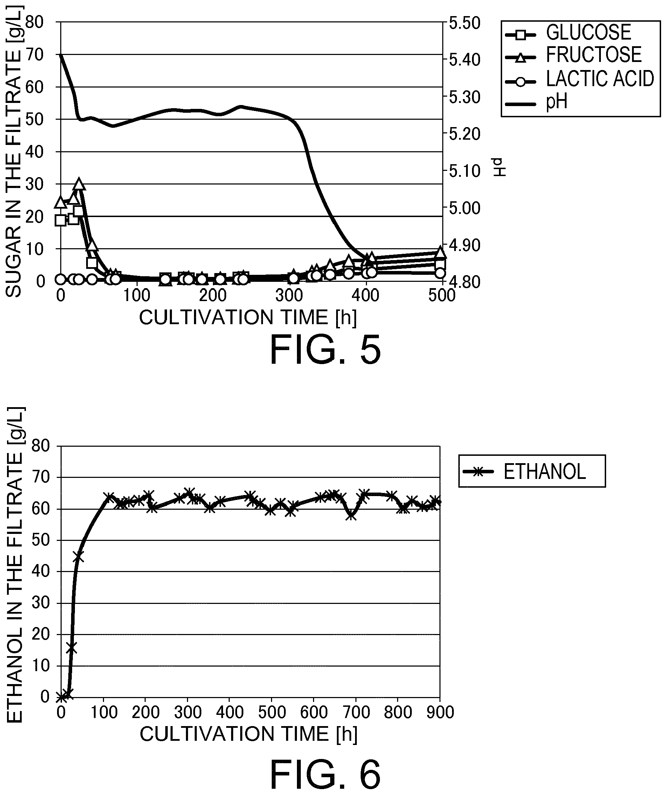

The results were as shown in FIG. 5. The concentrations of glucose and fructose derived from the fermentation raw material increased after 300 hours from the start of the test as in the Example 1. The concentration of lactic acid also increased after 300 hours from the start of the test as did the concentrations of glucose and fructose. Further, the pH value of the culture in the fermenter also decreased after 300 hours from the start of the test. From these results, despite sterilization of the region including the fermenter (3) enclosed in the dotted line (21), contamination was observed.

### Example 3: Fermentation with Routine Washing of Fermentation Raw Material Supply Lines

Fermentation was carried out using the continuous fermentation apparatus (1) shown in FIG. 2 in the same manner as in Example 2 except that the supply line (6) was disconnected and washed every 72 hours. The supply line (6) was washed until all sugars adhering to the supply line were washed away by passage of water. In the test, the concentration of ethanol in the filtrate was measured. As indices of contamination, the concentration of glucose (fermentation raw material), the concentration of fructose (fermentation raw material), and the concentration of lactic acid in the filtrate, and the pH value of the culture in the fermenter were measured.

As a result, the concentration of ethanol was as shown in FIG. 6. It was found that the concentration of ethanol in the filtrate did not decrease even after 300 hours, and the filtrate kept the concentration of ethanol at about 60 g/L over a period from 100 to 900 hours after the start of the test.

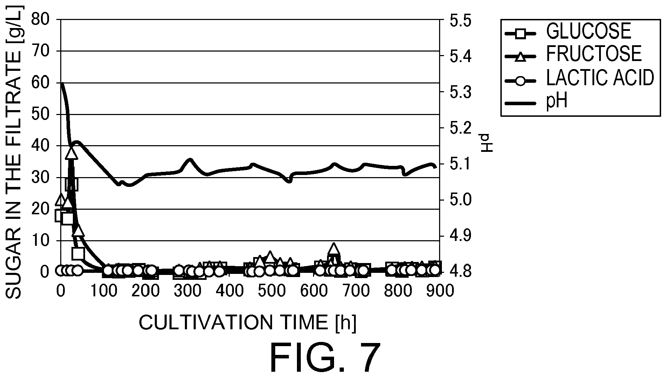

The concentration of glucose (fermentation raw material), the concentration of fructose (fermentation raw material), the concentration of lactic acid and the pH value of the culture in the fermenter were as shown in FIG. 7. The pH value of the culture in the fermenter also kept a constant value. In addition, over a period from 100 to 900 hours after the start of the test, all of the concentrations of glucose, fructose, and lactic acid in the filtrate kept almost zero.

From the above results, it was found that the supply line is the site that causes contamination and interferes continuous fermentation in the continuous fermentation apparatus comprising a separation membrane module. From these facts, it is found that a long-term continuous fermentation without contamination can be achieved by preparing a plurality of fermentation raw material supply systems comprising a fermentation raw material tank and a supply line, and using the plurality of fermentation raw material supply systems in rotation at a determined interval capable of preventing contamination that interferes continuous fermentation while washing the fermentation raw material supply system out of use repeatedly. The present inventors found that a long-term continuous fermentation set in the same test as in Example 3 can be carried out by the continuous fermentation apparatus shown in FIG. 1.

### DESCRIPTION OF SYMBOLS

- 1:: continuous fermentation apparatus
- 2a and 2b:: fermentation raw material supply system
- 3:: fermenter
- 4:: separation membrane module
- 5:: fermentation raw material tank
- 6:: supply line
- 7:: pump
- 8:: stirrer
- 9:: sugar concentration meter
- 10:: first line
- 11:: second line
- 12:: porous membrane
- 13:: third line
- 14:: washing liquid tank
- 14':: water tank
- 15 and 18:: line
- 16a, 16b, 19a, 19b, 20a and 20b:: connecting valve
- 17a and 17b:: line mixer
- 21:: sterilized area

## Claims

1. A method for producing an alcohol by continuous fermentation using a fermentation raw material,
the method comprising steps of:
culturing a microorganism in said fermentation raw material in a fermenter to obtain a fermented liquid, while supplying said fermentation raw material into said fermenter using a plurality of fermentation raw material supply systems;
filtering the obtained fermented liquid through a separation membrane module to obtain an alcohol-containing filtered liquid and an unfiltered liquid; and
retaining or refluxing the obtained unfiltered liquid in said fermenter;
wherein said plurality of fermentation raw material supply systems each independently comprise a fermentation raw material tank for storing said fermentation raw material which is the same as each other, and a supply line connecting said fermentation raw material tank and said fermenter; and
wherein said method comprises selecting a fermentation raw material supply system to be allowed to stop supplying said fermentation raw material in rotation from said plurality of fermentation raw material supply systems during the supply of said fermentation raw material using said plurality of fermentation raw material supply systems.

2. The production method according to claim 1, wherein said fermentation raw material is not sterilized.

3. The production method according to claim 1 or 2, wherein each of the fermentation raw material supply systems stops supplying said fermentation raw material each time the system supply said fermentation raw material for 1 to 6 days.

4. The production method according to any one of claims 1 to 3, wherein either of the fermentation raw material supply systems is washed while stopping the supply of said fermentation raw material.

5. The production method according to claim 4, wherein said fermentation raw material supply system stopping the supply of said fermentation raw material resumes the supply of said fermentation raw material after the washing.

6. The production method according to claim 4 or 5, comprising supplying a liquid obtained through the washing of said fermentation raw material supply system to the fermenter.

7. The production method according to any one of claims 1 to 6, wherein said alcohol is at least one selected from the group consisting of methanol, ethanol, propanol and butanol.

8. The production method according to any one of claims 1 to 7, wherein said fermentation raw material is molasses.

9. A continuous fermentation apparatus for producing an alcohol by continuous fermentation using a fermentation raw material, the apparatus comprising:
a plurality of fermentation raw material supply systems for supplying said fermentation raw materials which are the same as each other;
a fermenter in which a microorganism is cultured in said fermentation raw material supplied from said plurality of fermentation raw material supply systems to obtain a fermented liquid;
a separation membrane module for filtering the fermented liquid obtained in the fermenter;
a first line connecting said fermenter and said separation membrane module and delivering said fermented liquid obtained in said fermenter to said separation membrane module; and
a second line connecting said separation membrane module and said fermenter and delivering an unfiltered liquid obtained by filtration of said fermented liquid in said separation membrane module to said fermenter;
wherein said plurality of fermentation raw material supply systems each independently comprise a fermentation raw material tank for storing said fermentation raw material which contains sugars and is the same as each other, and a supply line connecting said fermentation raw material tank and said fermenter.

10. The continuous fermentation apparatus according to claim 9, wherein said supply line comprises a line mixer.

11. The continuous fermentation apparatus according to claim 9 or 10, wherein said fermentation raw material supply system does not comprise a sterilizer for said fermentation raw material.

12. The continuous fermentation apparatus according to any one of claims 9 to 11, wherein the apparatus further comprises washing liquid tanks each connected to each of said plurality of fermentation raw material supply systems.

13. The continuous fermentation apparatus according to claim 12, wherein one washing liquid tank is connected to said plurality of fermentation raw material supply systems.
